# EUROPEAN PATENT SPECIFICATION

(11) **EP 4 013 398 B1**
(45) Date of publication and mention of the grant of the patent: **31.12.2025**
(21) Application number: 20853716.7
(22) Date of filing: 05.08.2020
(51) Int. Cl.: A61K 31/138, C07C 17/16, C07C 41/16, C07C 213/00, C07C 213/08

(54) **AN IMPROVED PROCESS FOR PREPARATION OF VILANTEROL OR A PHARMACEUTICALLY ACCEPTABLE SALT THEREOF**
VERBESSERTES VERFAHREN ZUR HERSTELLUNG VON VILANTEROL ODER EINES PHARMAZEUTISCH ANNEHMBAREN SALZES DAVON
PROCÉDÉ AMÉLIORÉ POUR LA PRÉPARATION DE VILANTÉROL OU D'UN SEL PHARMACEUTIQUEMENT ACCEPTABLE DE CELUI-CI

(30) Priority: 16.08.2019 IN 201921033147
(43) Date of publication of application: 22.06.2022
(73) Proprietor: Melody Healthcare Pvt. Ltd., Mumbai 400021 (IN)
(72) Inventor: SINGHANIA, Harshvardhan, Mumbai, 400021 (IN); SINGHANIA, Devang, Mumbai, 400021 (IN)
(74) Representative: WP Thompson
(86) International application number: PCT/IN2020/050691
(87) International publication number: WO 2021/033198

(56) References cited:
- WO-A1-2017/001907
- WO-A2-2019/016511
- CN-A- 102 451 178
- US-A1- 2015 239 862
- US-A1- 2015 239 862
- "Crystalline forms of vilanterol base and vilanterol trifenatate", RESEARCH DISCLOSURE, KENNETH MASON PUBLICATIONS, HAMPSHIRE, UK, GB, vol. 604, no. 2, August 2014 (2014-08-01), pages 4, XP007143309, ISSN: 0374-4353

## Description

### Technical filed:

The invention relates to an improved process for preparation of Vilanterol or a pharmaceutically acceptable salt thereof with good yields and high purity.

### Background and prior art:

Various phenethanolamine derivatives including Vilanterol; process for their preparation; compositions containing them and their use in prophylaxis and treatment of respiratory diseases were disclosed in WO 2003/024439. Vilanterol is chemically described as 4-1(1R)-2-[6-{2-(2,6-dichlorobenzyl)oxy]ethoxy}hexyl)amino]-1-hydroxyethyl}-2-(hydroxymethyl) phenol, represented by Formula I, as shown below.

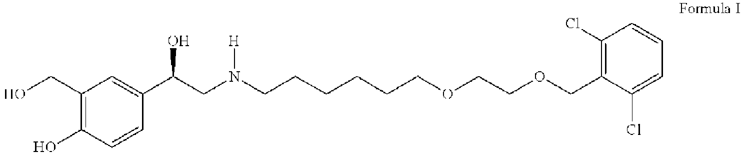

4-{(1R)-2-[(6-{2-[(2,6-dichlorobenzyl)oxy]ethoxy}hexyl)amino]-1-hydroxy ethyl}-2-(hydroxymethyl)phenol triphenylacetate salt is specifically described in WO2003/024439, in addition to other pharmaceutically acceptable salts thereof, such as acetate, α-phenylcinnamate, 1-naphthoate and (R)-mandelate salts.

WO2003/024439 and J. Med. Chem, 2010, 53, 4522-4530 discloses the process for preparation of Vilanterol along with pharmaceutically acceptable salt. The reaction sequence is shown below in scheme I:

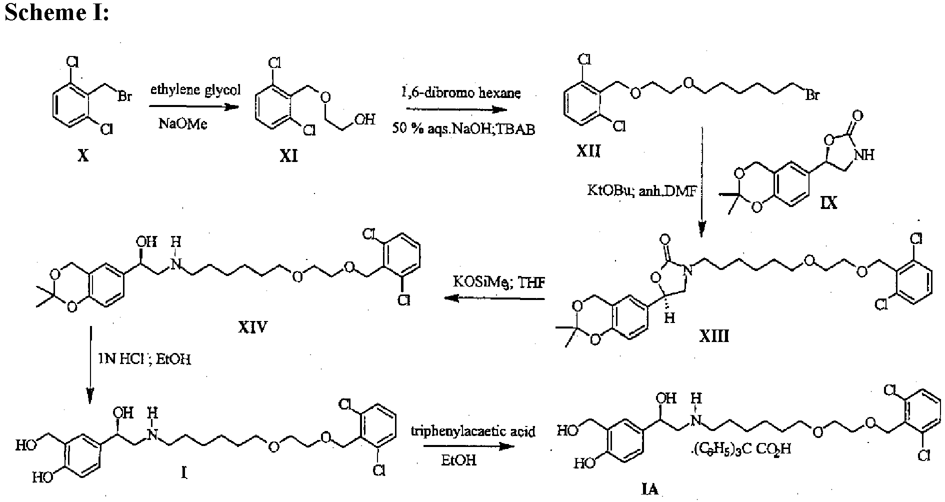

WO2003/024439 further describes a process for preparing (5R)-5-(2,2-dimethyl-4H-1,3-benzodioxin-6-yl)-1,3-oxazolidin-2-one, a key intermediate of Vilanterol.

The process is shown below in scheme II.

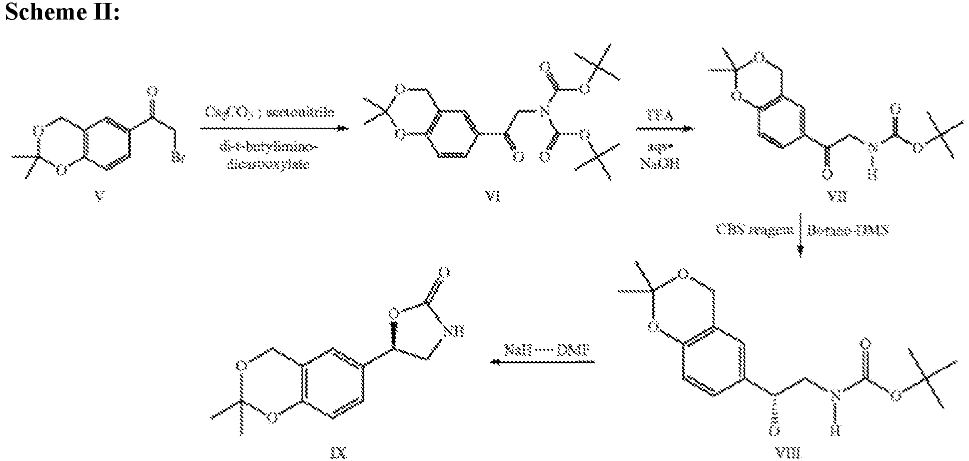

WO 2017/001907 discloses a process for preparing Vilanterol includes a bio catalytic conversion of a ketone substrate to its corresponding alcohol, and then converting the obtained alcohol into Vilanterol is disclosed.

Solid state forms of Vilanterol tartrate, preferably Vilanterol L-tartrate, in substantially pure form are also disclosed.

It is important that the chiral purity of the (5R)-5-(2,2-dimethyl-4H-1,3-benzodioxin-6-yl)-1,3-oxazolidin-2-one compound is essential, as the presence of the corresponding isomeric impurities in the chiral intermediate would carry forward up to final product, Vilanterol, which are difficult to separate and thus necessitates tedious purification of the final product. Therefore, it becomes necessary to prepare the key intermediate, (5R)-5-(2,2-dimethyl-4H-1,3-benzodioxin-6-yl)-1,3-oxazolidin-2-one compound in chirally pure form in order to make the final API free from the corresponding isomeric impurities and to make the process for production of Vilanterol commercially economical.

The prior arts demonstrates extensive use of column chromatography for all the intermediates and the final compounds viz., Vilanterol and its pharmaceutical salts and thus difficult to scale up for industrial production.

Accordingly, it is an objective of the present invention to develop a simple, industrially feasible and scalable process using easily available cheaper raw materials for the synthesis of Vilanterol with a purity of greater than 99% with known and unknown impurities less than 1%.

US2015239862A1 discloses a process for the preparation of vilanterol and pharmaceutically acceptable salts thereof, which includes a process for preparing intermediates for the preparation of vilanterol.

### Summary of the invention:

In line with the above objective, the present invention provides an improved process for preparation of vilanterol Trifenatate characterized in that;
a) Brominating 2,6 Dichlorophenyl methanol with a brominating agent at a temperature of 40-85°C to obtain 2-(Bromomethyl)-1,3-dichlorobenzene;
b) Reacting the 2-(Bromomethyl)-1,3-dichlorobenzene in ethylene glycol with Potassium salt of ethylene glycol at 40 to 70°C to obtain 2-(2, 6-dichlorobenzyloxy) ethanol;
c) Reacting the 2-(2, 6- dichlorobenzyloxy) ethanol with 1,6-Dibromohexane in toluene presence of KOH and Catalytic amount of Tetra butyl Ammonium Hydrogen Sulphate (TBAHS) at 40-65°C to obtain 2-((2-((6-bromohexyl)oxy)ethoxy)methyl)-1,3-dichlorobenzene;
d) Reacting the 2-((2-((6-bromohexyl)oxy)ethoxy)methyl)-1,3-dichlorobenzene with (5R)-5-(2,2-Dimethyl-4H-1,3-benzodioxin-6-yl)-1,3-oxazolidin-2-one in DMF at ambient temperature, followed by extracting the reaction mass into methylene dichloride to obtain crude (R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one;
e) Purifying the crude (R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one by treating with acetonitrile and hexane;
f) Cleaving the (5R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one with Potassium trimethyl silanolate in THF at 50-70°C to obtain (1R)-2-[(6-{2-[(2,6-dichlorobenzyl)oxy]ethoxy}hexyl)amino]-1-(2,2-dimethyl-4H-1,3-benzodioxin-6-yl)ethanol;
g) Deprotecting the ( 1R)-2-[(6-{2-[(2,6-dichlorobenzyl)oxy]ethoxy}hexyl)amino]-1-(2,2-dimethyl-4H-1,3-benzodioxin-6-yl)ethanol in presence of HCl in acetone to obtain Vilanterol followed by successive washings of Vilanterol with dil. acetic acid & diethyl ether;
h) Treating the Vilanterol with Tri phenyl acetic acid in acetone at 45-65°C to yield crude Vilanterol Trifenatate; and
i) Purifying the crude Vilanterol Trifenatate.

The brominating agent in step a) may be selected from Hydrobromic acid and Sulphuric acid; bromine, NBS etc.

In an embodiment, the Vilanterol is simply washed with dil. acetic acid & diethyl ether in a ratio of 1:4, to give Vilanterol with HPLC purity of more than 98%. The simple method of washing the crude Vilanterol with the combination of dil. acetic acid & diethyl ether avoids the need for chromatographic techniques unlike the prior arts.

The use of tetra butyl ammonium hydrogen sulphate (TBAHS) in catalytic amounts in the production of 2-((2-((6-bromohexyl)oxy)ethoxy)methyl)-1,3-dichlorobenzene increase rate of reaction with higher selectivity than tetra butyl ammonium bromide (TBAB) and thus reduces the formation of by-products and further avoids the environmental issues related to bromide ions in the case of TBAB.

In an embodiment, the purification of the crude (R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one is carried out by simply treating with acetonitrile and hexane in a ratio of 6:4, to obtain the product with a purity of more than 99%.

Described herein is also a process for purification of crude Vilanterol Trifenatate which comprises;
a) Dissolving the Vilanterol Trifenatate in acetone at a temperature of 50-55°C;
b) Cooling the reaction mass to room temperature; and
c) Adding anti-solvent consisting a mixture of acetonitrile, methylene dichloride and diethyl ether to precipitate pure Vilanterol Trifenatate in polymorphic form 1.

According to the process for purification of crude Vilanterol Trifenatate, the mixture of anti-solvent consists of acetonitrile, methylene dichloride and diethyl ether in a ratio of 2:3:1.

The Vilanterol Trifenatate thus obtained has purity of more than 99.5%. All known impurities are below 0.15% and all unknown impurities in the Vilanterol Trifenatate are below 0.10%.

Thus the process of the present invention is simple, cost effective and industrially scalable.

### Description of drawings:

Fig 1 shows the PXRD of the Vilanterol Trifenatate polymorphic form 1 obtained as per example 4.
Fig 2 shows DSC of the Vilanterol Trifenatate polymorphic form 1 obtained as per example 4.

### Detailed description:

The invention will now be described in detail in connection with certain preferred and optional embodiments, so that various aspects thereof may be more fully understood and appreciated.

Accordingly, the present invention provides an improved process for preparation of vilanterol and its pharmaceutical salts characterized in that;
a) Brominating 2,6 Dichlorophenyl methanol with a brominating agent at a temperature of 40-85°C to obtain 2-(Bromomethyl)-1,3-dichlorobenzene;
b) Reacting the 2-(Bromomethyl)-1,3-dichlorobenzene in ethylene glycol with Potassium salt of ethylene glycol at 40 to 70°C to obtain 2-(2, 6-dichlorobenzyloxy) ethanol;
c) Reacting the 2-(2, 6- dichlorobenzyloxy) ethanol with 1,6-Dibromohexane in toluene presence of KOH and Catalytic amount of Tetra butyl Ammonium Hydrogen Sulphate (TBAHS) at 40-65°C to obtain 2-((2-((6-bromohexyl)oxy)ethoxy)methyl)-1,3-dichlorobenzene;
d) Reacting the 2-((2-((6-bromohexyl)oxy)ethoxy)methyl)-1,3-dichlorobenzene with (5R)-5-(2,2-Dimethyl-4H-1,3-benzodioxin-6-yl)-1,3-oxazolidin-2-one in DMF at ambient temperature, followed by extracting the reaction mass into methylene dichloride to obtain crude (R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one;
e) Purifying the (R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one by treating with acetonitrile and hexane;
f) Cleaving the (5R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one with Potassium trimethyl silanolate in THF at 50-70°C to obtain (1R)-2-[(6-{2-[(2,6-dichlorobenzyl)oxy]ethoxy}hexyl)amino]-1-(2,2-dimethyl-4H-1,3-benzodioxin-6-yl)ethanol;
g) Deprotecting the 1R)-2-[(6-{2-[(2,6-dichlorobenzyl)oxy]ethoxy}hexyl)amino]-1-(2,2-dimethyl-4H-1,3-benzodioxin-6-yl)ethanol in presence of HCl in acetone to obtain Vilanterol followed by successive washings of Vilanterol with dil. acetic acid & diethyl ether;
h) Treating the Vilanterol with Tri phenyl acetic acid in acetone at 45-65°C to yield crude Vilanterol Trifenatate; and
i) Purifying the crude Vilanterol Trifenatate.

In a preferred embodiment, the stage wise details for process for preparation of Vilanterol Trifenatate is described herein below.

### Stage I:

### Part-A:

According to stage I, 2,6 Dichlorophenyl methanol (DCPM) undergoes Bromination in presence of Hydrobromic acid and Sulphuric acid at temperature of at 50-55°C for one hr. and then the temperature of the reaction mass is gradually increased to 70-75°C and maintained up to 4 hrs. to complete Bromination of DCPM. The Bromo compound of DCPM is further extracted into methylene dichloride and concentrated the methylene dichloride layer to obtain 2-(Bromomethyl)-1,3-dichlorobenzene, which is diluted with Ethylene Glycol and kept it for next reaction.

### Part-B:

Ethylene Glycol is reacted with Potassium tertiary butoxide at temperature 55 to 60°C to obtain Potassium salt of ethylene glycol, which is further cooled to 10-15°C.

### Part-C:

The Potassium salt of ethylene glycol obtained in Part-B is added to Part-A and maintained the reaction mass at 55-60°C for conversion of Bromo compound of DCPM into 2-(2, 6- dichlorobenzyloxy) ethanol. 2-(2, 6- dichlorobenzyloxy) ethanol thus obtained is then extracted by quenching the mass in Toluene+ Water, followed by concentration of the reaction mass in toluene layer to obtain 2-(2, 6-dichlorobenzyloxy) ethanol. The process is depicted in scheme III below.

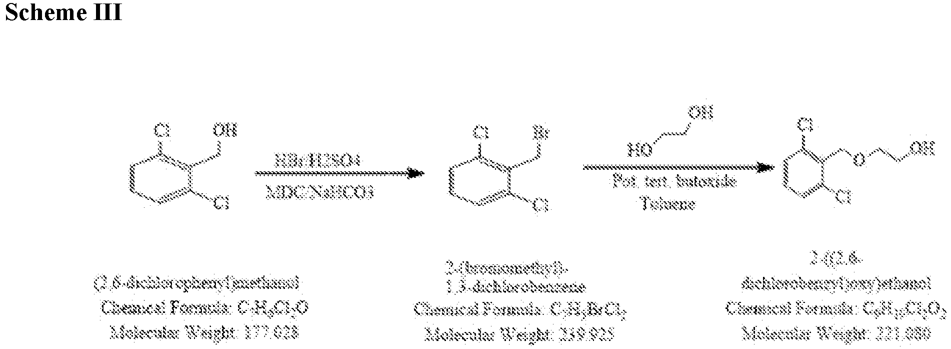

### Stage II:

In stage II, the 2-(2, 6- dichlorobenzyloxy) ethanol thus obtained from stage I is reacted with Potassium Hydroxide in presence of Toluene to form the Potassium salt of 2-(2, 6- dichlorobenzyloxy) ethanol, which further undergoes condensation reaction with 1,6-Dibromohexane in presence of Catalytic amount of Tetra butyl Ammonium Hydrogen Sulphate(TBAHS). The reaction is completed by maintaining the mass at 40-65°C for 8-24 hrs. The product is obtained in Toluene, which is further purified by water washing. Toluene layer is concentrated to obtain residue containing traces of unreacted 1,6-Dibromohexane, which is separated by high temperature vacuum distillation to obtain 2-((2-((6-bromohexyl)oxy)ethoxy)methyl)-1,3-dichlorobenzene in oily mass form. The process is depicted in scheme IV below.

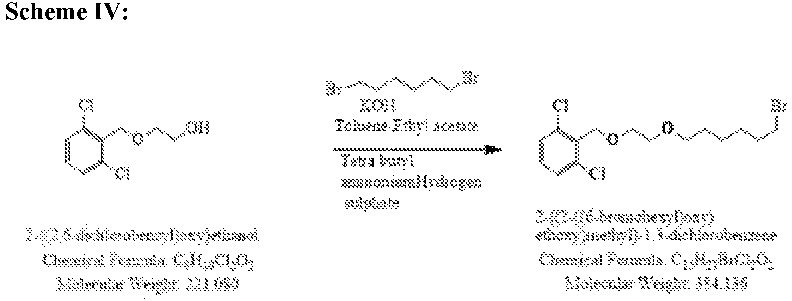

### Stage III:

In stage III, Potassium tert-butoxide is added to a solution of (5R)-5-(2,2-Dimethyl-4H-1,3-benzodioxin-6-yl)-1,3-oxazolidin-2-one (DMBO) in DMF under N2 and the reaction is stirred for 1 h at ambient temperature. A solution of 2-((2-((6-bromohexyl)oxy)ethoxy)methyl)-1,3-dichlorobenzene obtained in stage II taken in anhydrous DMF is added to the above reaction mass and the reaction mass is maintained under stirring at ambient temperature for 3hr. The reaction mixture is then diluted with ice/water and extracted with MDC. The organic layer is separated and washed successively with water. The solution is concentrated to dryness up to 50-55°C and the residue is dissolved in Acetonitrile and added Hexane in a ratio of 60:40. The reaction mass is heated up to the 55-60°C; slowly cooled to 35-40°C and separated the acetonitrile layer. The acetonitrile layer is washed with Hexane at 25-30°C. The acetonitrile layer is concentrated to dryness under vacuum at 50-55°C, slowly cooled reaction mass to obtain the residue (R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one with a purity> 99.8%.

In an alternate embodiment, the product may be eluted on column chromatography using acetonitrile and hexane in 60:40 ratio to obtain pure fractions of (R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one, which are obtained with a purity> 99.8%.

(R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one thus obtained is dissolved in THF at ambient temperature and reacted with Potassium trimethyl silanolate at 50-70°C to obtain (1R)-2-[(6-{2-[(2,6-dichlorobenzyl)oxy]ethoxy}hexyl)amino]-1-(2,2-dimethyl-4H-1,3-benzodioxin-6-yl)ethanol. The process of stage III is depicted in scheme V.

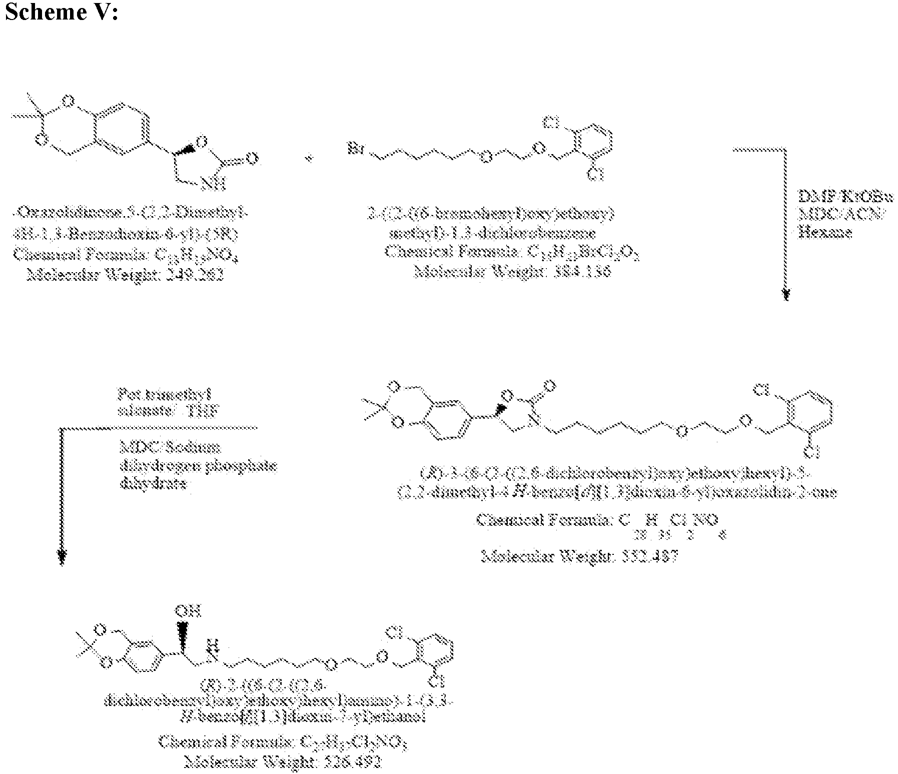

### Stage IV

According to stage IV, (1R)-2-[(6-{2-[(2,6-dichlorobenzyl)oxy]ethoxy}hexyl)amino]-1-(2,2-dimethyl-4H-1,3-benzodioxin-6-yl)ethanol thus obtained from stage III is dissolved in acetone under nitrogen at ambient temperature. The reaction mass is cooled to 0-5°C and 0.5N HCl is added slowly under stirring over one hour period, to obtain crude Vilanterol. The Vilanterol thus obtained is given successive washings with dil. acetic acid & diethyl ether to improve the purity of the Vilanterol. These washings gives Vilanterol with HPLC purity of more than 98% and thus the need for chromatography unlike the prior art is avoided.

Vilanterol thus obtained is dissolved in Acetone followed by treatment with Tri phenyl acetic acid at 45-65°C to give crude Vilanterol Trifenatate. The process of stage IV is depicted in scheme VI.

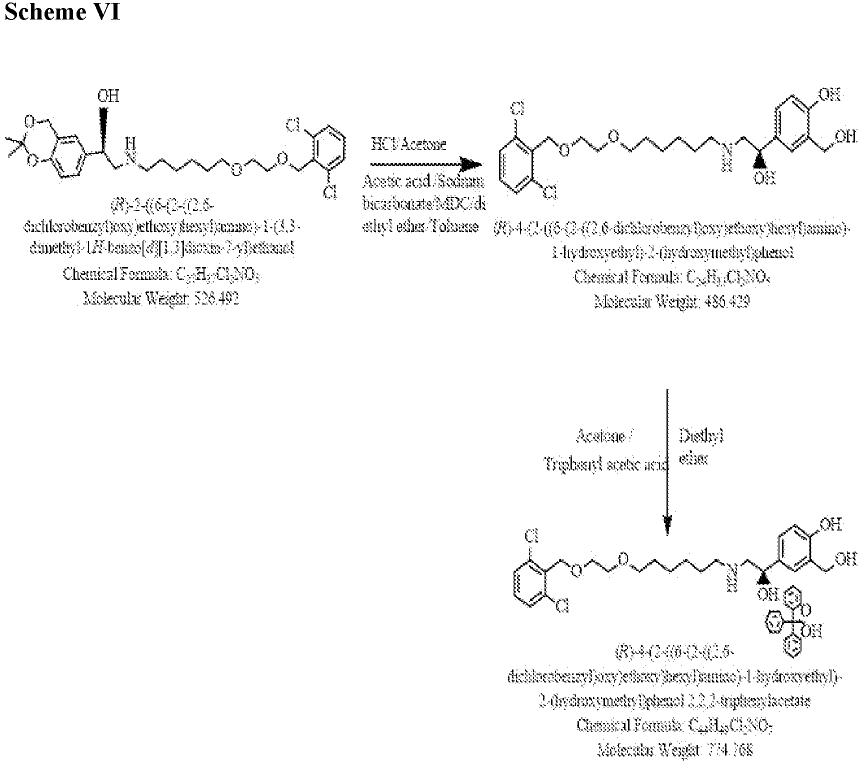

### Stage V:

The crude Vilanterol Trifenatate is dissolved in Acetone at temperature of 50-55°C.

The reaction mass is cooled to room temperature and an anti-solvent consisting of acetonitrile, dimethyl ether and MDC are added to precipitate pure Vilanterol Trifenatate. The process of stage V is depicted in scheme VII.

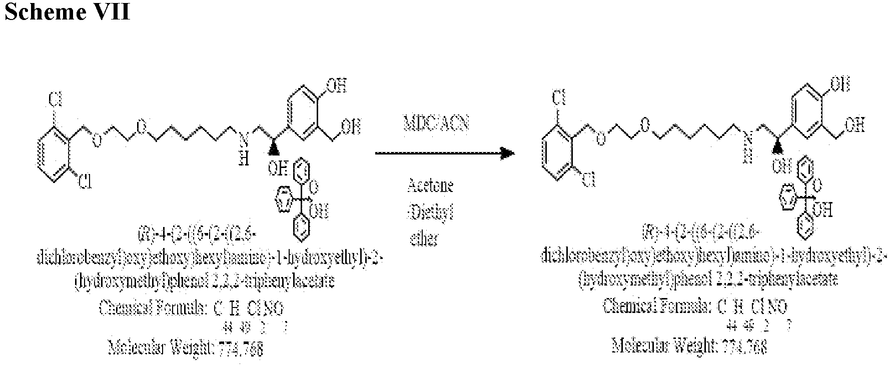

The present invention is exemplified by the following examples which are provided for illustration purpose only and, should not be construed to limit the scope of the invention.

### EXAMPLES:

### Example 1

### Stage I:

### Preparation of 2-(Bromomethyl)-1,3-dichlorobenzene

Charged Hydrobromic acid 48.0% (240.0 gm., 1.26 eqt) at 25-30°C, cooled the reaction mass to 20 to 25°C and added slowly sulphuric acid (100.0 gm., (0.91 eqt) at 20-25°C temperature. Stirred & maintained the reaction mass for 5 to 10 min at 20 to 25°C. Added slowly (2,6-dichlorophenyl)methanol (DCPM)(200.0 gm., 1.00 eqt) at 20 to 25°C temperature. Stirred & maintained the reaction mass for 4.0 hrs. at 70-75°C temperature. Cooled the reaction mass to 25 to 30°C and charged MDC (1000ml, 5.0 V) stirred and separated MDC layer at 25 to 30°C temperature. Collected the MDC layer & treated with aq. Sodium bicarbonate solution (40 gm) in purified Water (1000ml) at 25 to 30°C. MDC layer was taken in a clean RBF at 25 to 30°C, distilled MDC layer at atmospheric pressure at 45°C. Cooled oily mass to 25 to 30°C, added ethylene glycol (200 ml) at 25 to 30°C, stirred the reaction mass for 5 to 10 min at 25 to 30°C temperature to obtain 2-(Bromomethyl)-1,3-dichlorobenzene.

### Preparation of 2-(2, 6- dichlorobenzyloxy) ethanol

Potassium tert-butoxide (100gm) was added portion wise to ethylene glycol (1200ml) under nitrogen keeping the temperature below 35°C. The temperature of the reaction mass was raised to 40-45° C, and stirred for one hour period. (2-(Bromomethyl)-1,3-dichlorobenzene) obtained in the previous stage was charged to the above reaction mass at 40-45° C and the mixture was heated to 55-60° C, for 1 h. After completion of the reaction, the mass was cooled to 20° C, water was added and the mixture was extracted with toluene (1000). The aqueous layer was separated and extracted twice with toluene. The organic extracts were combined and then evaporated to dryness to afford the desired compound as colorless oil (229.00 gm.)
Yields: 92.3%.
Purity by HPLC: 93.67%

### Example 2

### Stage II:

### Preparation of 2-((2-((6-bromohexyl)oxy)ethoxy)methyl)-1,3-dichlorobenzene

KOH (127gm, 2.13 eqt), 2-(2,6-dichlorobenzyloxyl)ethanol (225gm 1.0eqt), 1,6-dibromohexane (493.00 gm., 2.0 eqt;) and Tetra butyl ammonium Hydrogen Sulphate (3.75 gm., 0.01eqt) in toluene (450.00 ml, 2V) were heated to 55-60° C, for 8-20 hrs. Upon completion, the reaction mass was diluted with water and toluene under cooling. The aqueous phase was separated and diluted with water then back extracted with toluene. The combined toluene extracts were washed twice with water and then evaporated to dryness under vacuum to afford the crude compound. The excess Di bromo hexane was removed by vacuum distillation to get brown color oil as crude, which was directly used as such in the next step. (310.00 gm.).
Yield: 79.60%;
Purity of the crude by HPLC: 88.50%

### Example 3

### Stage III

### Preparation of Vilanterol Trifenatate

### Step (1):

### Preparation of (R)-3-(6-{2-[(2, 6-dichlorobenzyl)oxy]ethoxy}hexyl)-5-(2,2-dimethyl-4H-1,3-benzodioxin-6-yl)-1,3-oxazolidin-2-one

Potassium tert-butoxide (51.600 gm,0.57eqt) was added to a solution of (R)-5-(2,2-dimethyl-4H-benzo[d][1,3]dioxin-6-yl)oxazolidin-2-one, DMBO(200.00 gm,1.0eqt) in DMF (1000.00 ml,5V) under N2 and the reaction stirred for 1 hr. at ambient temperature. A solution of 2-((2-((6-bromohexyl)oxy)ethoxy)methyl)-1,3-dichlorobenzene (307.00 gm,1.0eqt) in anhydrous DMF (200.00 ml,1.0V) was added to the above reaction mass and the reaction was stirred at ambient temperature for 3 hr. The reaction mixture was diluted with ice/water and extracted with MDC. The organic layer was separated then washed successively with water/saturated brine and dried over sodium sulfate. The solution was concentrated to dryness and the residue thus obtained was purified by column chromatography by eluting with acetonitrile and hexane (60:40). The pure fractions were concentrated under vacuum to afford pure (R)-3-(6-(2-((2,6-dichlorobenzyl)oxy)ethoxy)hexyl)-5-(2,2-dimethyl-4H-benzo[d][1,3]dioxin-6-yl)oxazolidin-2-one, as pale yellow colour oil (430.00gms).
Yield: 97.50%
HPLC Purity: 99.5%
Chiral Purity, R-isomer: 99.8%

In a process variant, the reaction mixture is diluted with ice/water and extracted with MDC. The organic layer was separated and washed successively with water. The solution was concentrated to dryness up to 50-55°C and the residue was dissolved in Acetonitrile and added Hexane in a ratio of 60:40. The reaction mass was heated up to the 55-60°C; slowly cooled to 35-40°C and separated the acetonitrile layer. The acetonitrile layer was washed with Hexane at 25-30°C; concentrated to dryness under vacuum at 50-55°C and slowly cooled reaction mass to obtain the residue (R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one, as pale yellow colour oil (430.00gms).
Yield: 97.50%
HPLC Purity: 99.5%
Chiral Purity, R-isomer: 99.7%

### Step (2):

### Preparation of (1R)-2-[(6-{2-[(2,6-dichlorobenzyl)oxy]ethoxy}hexyl)amino]-1-(2,2-dimethyl-4H-1,3-benzodioxin-6-yl)ethanol

(R)-3-(6-(2-((2,6-dichlorobenzyl)oxy)ethoxy)hexyl)-5-(2,2-dimethyl-4H-benzo[d][1,3]dioxin-6-yl)oxazolidin-2-one from the previous stage was dissolved in THF (1000.00 ml,5.0V) at ambient temperature. Potassium trimethyl silanolate (190.00 gm., 1.85eqt) was added to the above reaction mass at ambient temperature under nitrogen. The temperature of the reaction mass was raised to 60-65° C, and stirred for period of an hour. After completion of the reaction, the reaction mass was cooled to 5-10° C, treated with 5% dihydrogen phosphate dihydrate (pH: 6-7) and extracted with ethyl acetate. The organic layer was separated and washed successively with water/saturated brine and dried over sodium sulfate. The solution was concentrated to dryness under vacuum to obtain the residue. The pure fractions were concentrated under vacuum to afford (R)-2-((6-(2-((2,6-dichlorobenzyl)oxy)ethoxy)hexyl)amino)-1-(2,2-dimethyl-4H-benzo[d][1,3]dioxin-6-yl)ethanol, as pale yellow color oil.

Then (R)-2-((6-(2-((2,6-dichlorobenzyl)oxy)ethoxy)hexyl)amino-1-(2,2-dimethyl-4H-benzo[d][1,3]dioxin-6-yl)ethanol was dissolved in acetone (1000.00 ml,5V) under nitrogen at ambient temperature. The reaction mass was cooled to 0-5° C and 0.5N HCl (1000.00 ml, 5V) was added slowly. The reaction mass was allowed to stir for completion over a period of one hour. The reaction mass was diluted with dichloromethane and water, followed by addition of saturated sodium bicarbonate solution (10 V) at 0-5° C. The organic layer was separated then washed successively with dil. acetic acid & diethyl ether in a ratio of 1:4 respectively and dried over sodium sulfate. The solution was concentrated to dryness under vacuum to afford (R)-4-(2-((6-(2-((2,6-dichlorobenzyl)oxy)ethoxy)hexyl)amino)-1-hydroxyethyl)-2-(hydroxymethyl)phenol, as pale yellow colour oil (307.00 gm).
Yield:-79.30%
Purity:-98.50%

### Step (3):

### Preparation of Vilanterol Trifenatate

Triphenyl acetic acid (72.50 gm,0.31eqt) was added to a solution of (R)-4-(2-((6-(2-((2,6-dichlorobenzyl)oxy)ethoxy)hexyl)amino)-1-hydroxyethyl)-2-(hydroxymethyl)phenol (307gms, 1eq) in acetone (2000.00 ml,10V) at ambient temperature and the mixture heated to 50-55° C to obtain a homogenous solution. The mixture was allowed to cool to ambient temperature; the resultant product was filtered, washed with chilled acetone, dried under vacuum at 50° C, to afford (R)-4-(2-((6-(2-((2,6-dichlorobenzyl)oxy)ethoxy)hexyl)amino)-1-hydroxyethyl)-2-(hydroxymethyl)phenol 2,2,2-triphenylacetate as a white solid (415 gms).
Yield: 85%
Purity by HPLC: NLT 98.50%
All known impurities are NMT 0.20%
All unknown impurities NMT 0.15%
Chiral purity-R-isomer: NLT 99.85%

### Reference Example 4

### Stage IV:

### Purification of Vilanterol Trifenatate

### (R)-4-(2-((6-(2-((2,6-dichlorobenzyl)oxy)ethoxy)hexyl)amino)-1-hydroxyethyl)-2-(hydroxymethyl)phenol 2,2,2-triphenylacetate(PURE)

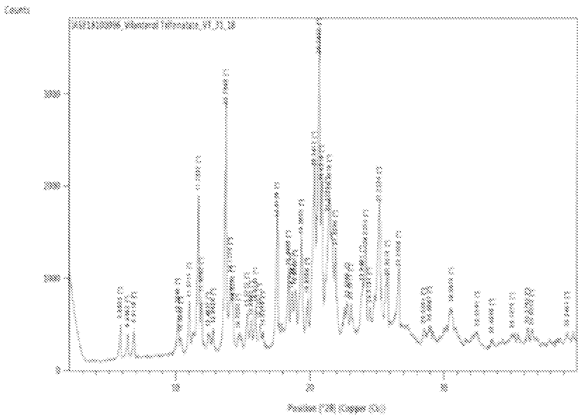

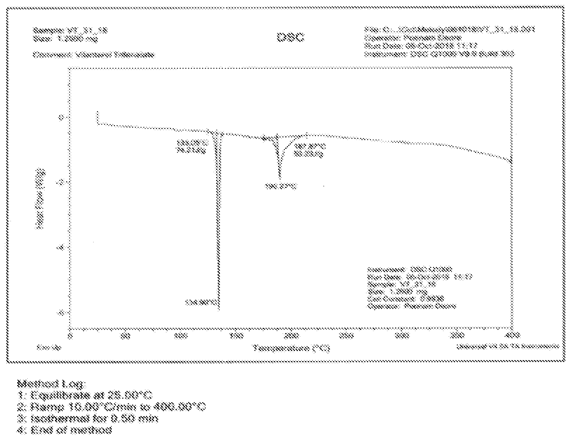

(R)-4-(2-((6-(2-((2,6-dichlorobenzyl)oxy)ethoxy)hexyl)amino)-1-hydroxyethyl)-2-(hydroxymethyl)phenol 2,2,2-triphenylacetate (Vilanterol Trifenatate-crude) was dissolved in Acetone (4 V) at a temperature of 50-55° C. Cooled reaction mass to room temperature followed by added a mixture of acetonitrile (1V) and MDC (1.50 V) & diethyl ether (0.50 V) to precipitate the product and washed the product with the same solvent mixture to obtain the pure Vilanterol Trifenatate. The PXRD and DSC of the Vilanterol Trifenatate thus obtained were shown in figures 1 and 2 respectively.
Yield: 70 %
Purity by HPLC: NLT 99.00%
All known impurities are NMT 0.15%
All unknown impurities below 0.10%
Chiral purity-R-isomer: NLT 99.85%
S-isomer: NMT 0.10%
1.18 RRT Specified known impurity NMT 0.30%

The improved process for the preparation of Vilanterol Trifenatate as demonstrated in the present invention is useful for industrial scale up while retaining the chemical and chiral purity of the product.

## Claims

1. An improved process for preparation of vilanterol Trifenatate **characterized by** the following steps;
a) Brominating 2,6 Dichlorophenyl methanol with a brominating agent at a temperature of 40-85°C to obtain 2-(bromomethyl)-1,3-dichlorobenzene;
b) Reacting the 2-(bromomethyl)-1,3-dichlorobenzene in ethylene glycol with Potassium salt of ethylene glycol at 40 to 70°C to obtain 2-(2, 6- dichlorobenzyloxy) ethanol;
c) Reacting the 2-(2, 6- dichlorobenzyloxy) ethanol with 1,6-Dibromohexane in toluene presence of KOH and Catalytic amount of Tetra butyl Ammonium Hydrogen Sulphate (TBAHS) at 40-65°C to obtain 2-((2-((6-bromohexyl)oxy)ethoxy)methyl)-1,3-dichlorobenzene;
d) Reacting the 2-((2-((6-bromohexyl)oxy)ethoxy)methyl)-1,3-dichlorobenzene with (SR)-5-(2,2-Dimethyl-4H-1,3-benzodioxin-6-yl)-1,3-oxazolidin-2-one (DMBO) in DMF at ambient temperature, followed by extracting the reaction mass into methylene dichloride to obtain crude (5R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one;
e) Purifying the (R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one by treating with acetonitrile and hexane;
f) Cleaving the (5R)-3-(6-{2-[(2,6-dichlorobenzyl)oxy]ethoxy}hexyl)-5-(2,2-dimethyl-4H-1,3-benzodioxin-6-yl)-1,3-oxazolidin-2-one with Potassium trimethyl silanolate in THF at 50-70°C to obtain (1R)-2-[(6-{2-[(2,6-dichlorobenzyl)oxy]ethoxy}hexyl)amino]-1-(2,2-dimethyl-4H-1,3-benzodioxin-6-yl)ethanol;
g) Deprotecting the 1R)-2-[(6-{2-[(2,6-dichlorobenzyl)oxy]ethoxy}hexyl)amino]-1-(2,2-dimethyl-4H-1,3-benzodioxin-6-yl)ethanol in presence of HCl in acetone to obtain Vilanterol followed by successive washing of Vilanterol with dil. acetic acid & diethyl ether;
h) Treating the Vilanterol with Tri phenyl acetic acid in acetone at 45-65°C to yield crude Vilanterol Trifenatate; and
i) Purifying the crude Vilanterol Trifenatate.

2. The process as claimed in claim 1, wherein, the brominating agent in step a) may be selected from Hydrobromic acid and Sulphuric acid; bromine, NBS.

3. The process as claimed in claim 1, wherein, the acetonitrile to hexane ratio is 6:4.

4. The process as claimed in claim 1, wherein, the dil. acetic acid & diethyl ether used for washing the Vilanterol is in a ratio of 1:4.

## Patentansprüche

1. Verbessertes Verfahren zur Herstellung von Vilanterol-Trifenatat, das durch die folgenden Schritte gekennzeichnet ist:
a) Bromieren von 2,6-Dichlorphenylmethanol mit einem Bromierungsmittel bei einer Temperatur von 40-85 °C, um 2-(Brommethyl)-1,3-dichlorbenzol zu erhalten;
b) Reagieren des 2-(Brommethyl)-1,3-dichlorbenzols in Ethylenglycol mit dem Kaliumsalz von Ethylenglycol bei 40 bis 70 °C, um 2-(2,6-Dichlorbenzyloxy)ethanol zu erhalten;
c) Reagieren des 2-(2,6-Dichlorbenzyloxy)ethanols mit 1,6-Dibromhexan in Toluol in Gegenwart von KOH und katalytischer Menge von Tetrabutylammoniumhydrogensulfat (TBAHS) bei 40-65 °C, um 2-((2-((6-Bromhexyl)oxy)ethoxy)methyl)-1,3-dichlorbenzol zu erhalten;
d) Reagieren des 2-((2-((6-Bromhexyl)oxy)ethoxy)methyl)-1,3-dichlorbenzols mit (5R)-5-(2,2-Dimethyl-4H-1,3-benzodioxin-6-yl)-1,3-oxazolidin-2-on (DMBO) in DMF bei Umgebungstemperatur, gefolgt von Extrahieren der Reaktionsmasse in Methylendichlorid, um rohes (5R)-3-(6-{2-[(2,6-Dichlorbenzyl)oxy]ethoxy}hexyl)-5-(2,2-dimethyl-4H-1,3-benzodioxin-6-yl)-1,3-oxazolidin-2-on zu erhalten;
e) Aufreinigen des (R)-3-(6-{2-[(2,6-Dichlorbenzyl)oxy]ethoxy}hexyl)-5-(2,2-dimethyl-4H-1,3-benzodioxin-6-yl)-1,3-oxazolidin-2-ons durch Behandeln mit Acetonitril und Hexan;
f) Spalten des (5R)-3-(6-{2-[(2,6-Dichlorbenzyl)oxy]ethoxy}hexyl)-5-(2,2-dimethyl-4H-1,3-benzodioxin-6-yl)-1,3-oxazolidin-2-ons mit Kaliumtrimethylsilanolat in THF bei 50-70 °C, um (1R)-2-[(6-{2-[(2,6-Dichlorbenzyl)oxy]ethoxy}hexyl)amino]-1-(2,2-dimethyl-4H-1,3-benzodioxin-6-yl)ethanol zu erhalten;
g) Entschützen des (1R)-2-[(6-{2-[(2,6-Dichlorbenzyl)oxy]ethoxy}hexyl)amino]-1-(2,2-dimethyl-4H-1,3-benzodioxin-6-yl)ethanols in Gegenwart von HCl in Aceton, um Vilanterol zu erhalten, gefolgt von aufeinander folgendem Waschen von Vilanterol mit verd. Essigsäure & Diethylether;
h) Behandeln des Vilanterols mit Triphenylessigsäure in Aceton bei 45-65 °C, um rohes Vilanterol-Trifenatat zu ergeben; und
i) Aufreinigen des rohen Vilanterol-Trifenatats.

2. Verfahren nach Anspruch 1, wobei das Bromierungsmittel im Schritt a) aus Bromwasserstoffsäure und Schwefelsäure, Brom, NBS ausgewählt sein kann.

3. Verfahren nach Anspruch 1, wobei das Verhältnis von Acetonitril zu Hexan 6 : 4 beträgt.

4. Verfahren nach Anspruch 1, wobei die verd. Essigsäure & Diethylether, die zum Waschen des Vilanterols verwendet werden, in einem Verhältnis von 1 : 4 vorliegen.

## Revendications

1. Procédé amélioré destiné à la préparation de trifénatate de vilantérol, **caractérisé par** les étapes suivantes :
a) la bromation de 2,6-dichlorophénylméthanol avec un agent de bromation à une température de 40-85°C pour obtenir du 2-(bromométhyl)-1,3-dichlorobenzène ;
b) la réaction du 2-(bromométhyl)-1,3-dichlorobenzène dans de l'éthylène glycol avec un sel de potassium de l'éthylène glycol à une température allant de 40 à 70°C pour obtenir du 2-(2,6-dichlorobenzyloxy)éthanol ;
c) la réaction du 2-(2,6-dichlorobenzyloxy)éthanol avec du 1,6-dibromohexane dans du toluène, en présence de KOH et d'une quantité catalytique d'hydrogénosulfate de tétrabutylammonium (TBAHS) à 40-65°C pour obtenir du 2-((2-((6-bromohexyl)-oxy)éthoxy)méthyl)-1,3-dichlorobenzène ;
d) la réaction du 2-((2-((6-bromohexyl)oxy)éthoxy)méthyl)-1,3-dichlorobenzène avec de la (5R)-5-(2,2-diméthyl-4H-1,3-benzodioxin-6-yl)-1,3-oxazolidin-2-one (DMBO) dans du DMF à température ambiante, suivie d'une extraction de la masse réactionnelle dans du dichlorométhane pour obtenir de la (5R)-3-(6-{2-[(2,6-dichlorobenzyl)oxy]éthoxy}hexyl)-5-(2,2-diméthyl-4H-1,3-benzodioxin-6-yl)-1,3-oxazolidin-2-one brute ;
e) la purification de la (R)-3-(6-{2-[(2,6-dichlorobenzyl)oxy]éthoxy}hexyl)-5-(2,2-diméthyl-4H-1,3-benzodioxin-6-yl)-1,3-oxazolidin-2-one par un traitement à l'acétonitrile et à l'hexane ;
f) le clivage de la (5R)-3-(6-{2-[(2,6-dichlorobenzyl)oxy]éthoxy}hexyl)-5-(2,2-diméthyl-4H-1,3-benzodioxin-6-yl)-1,3-oxazolidin-2-one avec du triméthylsilanolate de potassium dans du THF à 50-70°C pour obtenir du (1R)-2-[(6-{2-[(2,6-dichlorobenzyl)oxy]éthoxy}hexyl)amino]-1-(2,2-diméthyl-4H-1,3-benzodioxin-6-yl)éthanol ;
g) la déprotection du (1R)-2-[(6-{2-[(2,6-dichlorobenzyl)oxy]éthoxy}hexyl)amino]-1-(2,2-diméthyl-4H-1,3-benzodioxin-6-yl)éthanol en présence de HCl dans de l'acétone pour obtenir du vilantérol, suivie d'une lavage successif du vilantérol avec de l'acide acétique dilué et de l'éther diéthylique ;
h) le traitement du vilantérol par de l'acide triphénylacétique dans de l'acétone à 45-65°C pour obtenir du trifénatate de vilantérol brut ; et
i) la purification du trifénatate de vilantérol brut.

2. Procédé selon la revendication 1, dans lequel l'agent de bromation de l'étape a) peut être choisi parmi l'acide bromhydrique et l'acide sulfurique ; le brome, le NBS.

3. Procédé selon la revendication 1, dans lequel le rapport de l'acétonitrile à l'hexane est de 6:4.

4. Procédé selon la revendication 1, dans lequel l'acide acétique dilué et l'éther diéthylique utilisés pour le lavage du vilantérol se trouvent selon un rapport de 1:4.
